# EUROPEAN PATENT APPLICATION

(11) **EP 4 620 504 A2**
(43) Date of publication of application: **24.09.2025**
(21) Application number: 25166296.1
(22) Date of filing: 14.07.2021
(51) Int. Cl.: A61M 25/00

(54) **INTRALUMINAL PHYSIOLOGY SENSING DEVICE WITH EMBEDDED CONFORMAL CONDUCTORS**

(30) Priority: 15.07.2020 US 202063051927 P
(62) Divisional of application: 21749542.3
(71) Applicant: Koninklijke Philips N.V., 5656 AG Eindhoven (NL); Philips Image Guided Therapy Corporation, San Diego CA 92130 (US)
(72) Inventor: MAY, Justin Patrick, Eindhoven (NL); REYES, Ramiro, Eindhoven (NL); BURKETT, David, Eindhoven (NL)
(74) Representative: Philips Intellectual Property & Standards

(57) **Abstract**

An intraluminal sensing device includes a guidewire configured to be positioned within a body lumen of a patient. The guidewire includes a core wire, a first insulative coating covering at least a portion of a circumference of the core wire, and at least two conductive traces insulated from one another and extending longitudinally along at least the portion of the length of the core wire over an outer surface of the first insulative coating. Each conductive trace conforms to the curvature of the first insulative coating. The guidewire also includes a second insulative coating covering the conductive traces along at least the portion of the length of the core wire, and a sensor in electrical communication with the at least two conductive traces and configured to obtain physiological data while positioned within the body lumen. The guidewire also includes a proximal connector in electrical communication with the conductive traces.

## Description

### TECHNICAL FIELD

The subject matter described herein relates to intraluminal physiology sensing devices. For example, intravascular catheter or guidewires can include embedded, conformal metal conductors, and associated systems and methods.

### BACKGROUND

Intraluminal physiology sensing devices may be introduced into a body lumen of a patient, and may for example include physiological sensors at a distal end of a catheter or guidewire. Electrical wires may be employed to couple sensing elements at the distal end of the catheter or guidewire with a connector at a proximal end of the catheter or guidewire. Fine-gauge electrical wires may be referred to as filars. Small-diameter medical devices such as intraluminal (e.g., intravascular) catheters and guidewires may incorporate sensors (e.g., pressure, temperature, flow, or imaging sensors) whose power and communications occur through multi-filar (e.g., bifilar, trifilar, etc.) electrical conductor bundle or flat metal ribbons. In both cases, decreases in device size drive challenges in the device manufacturing process related to routing, wrapping, and securing of electrical conductors.

Guide wires and catheters are typically constructed with fine gauge filars and/or flattened ribbon wires that enable the transmission of electrical signal between electrical components within the device e.g. sensors, transducers and contacts. In order to make these connections, the filars and/or flattened ribbon wires may be embedded into polymers along the length of the device via wrapping or pulling processes on continuous reel-to-reel coating equipment. A bundle of electrical filars, may be joined as two, three, or more fine-gauge conductive filars, to make electrical connections between electrical components, e.g. sensors or transducers near the distal end of the device, and electrical contacts near the proximal end of the device. In order to make these connections, the filars or ribbons may be wrapped or pulled along the length of the device, from one end of the device to the other end. Because copper and many copper alloys have a low tensile and yield strength, the mechanical strength of electrical conductor materials is typically low. The manufacturing process for an intraluminal catheter or guidewire device may involve wrapping or stretching of the ribbon or multi-filar conductor bundle, which requires tensioning. Unfortunately, this tensioning can cause the conductive filars or ribbons to plasticly deform, which can result in severe and undesirable elongation and/or necking, e.g., up to 100% elongation in some current processes. The associated narrowing of the conductor affects both the mechanical strength and the maximum electrical current capacity of the filar, which can lead to manufacturing defects.

Some presently used devices employ flat, conductive metal ribbons that can have comparable current carrying capacity to filars, with a greater width and lesser height, potentially enabling smaller-diameter devices. These conductive ribbons may in some cases be better able to withstand tensioning, but do not conform to the cylindrical shape of a core wire, and thus may employ a thicker insulating layer to avoid exposed conductor at the edges of the ribbon. These ribbons are often manufactured separately and must be added to the device. However, positioning and maintaining them in a straight arrangement is challenging.

The information included in this Background section of the specification, including any references cited herein and any description or discussion thereof, is included for technical reference purposes only and is not to be regarded as subject matter by which the scope of the disclosure is to be bound.

### SUMMARY

Disclosed are intraluminal physiology sensing devices that include metal ink conductor assemblies. In some aspects, a metal ink conductor assembly can provide a single-layer or multi-layer application of conductive metal ink traces which permit improved electrical/mechanical performance and reduce or eliminate manufacturing issues associated with conductive filars or ribbons. Using nano-metal ink traces enables the use of conductive materials with lower tensile strengths and larger conductor cross-sections or surface areas. This may provide for lower electrical resistance/impedance along the length of the electro-mechanical device, as well as improving the straightness and torque response of intraluminal devices, while reducing manufacturing defects and reducing the complexity of the intraluminal device manufacturing process.

A nano-metal ink may be applied as a liquid coating directly onto an insulating polymer surrounding the core wire. Each layer of ink can then be sintered, which turns the coating into a solid metal with material properties equivalent to bulk materials. After sintering, an additional insulation layer may be coated to a desired thickness. When the desired thickness is achieved, the nano-metal layer or layers can then be individuated to create traces with the desired cross-sectional area.

The metal ink conductor assembly disclosed herein has particular, but not exclusive, utility for intraluminal medical catheters and guidewires.

One general aspect an intraluminal sensing device. The intraluminal sensing device includes a guidewire configured to be positioned within a body lumen of a patient, where the guidewire includes: a core wire; a first insulative coating covering at least a portion of a circumference of the core wire along at least a portion of a length of the core wire; at least two conductive traces, each including a thickness and a width and extending longitudinally along at least the portion of the length of the core wire over an outer surface of the first insulative coating, where each conductive trace includes a cross-sectional shape conforming to a curvature of the first insulative coating, where the at least two conductive traces and the core wire are insulated from one another; and a second insulative coating covering an outer surface of the at least two conductive traces along at least the portion of the length of the core wire; a sensor configured to obtain physiological data while positioned within the body lumen, where the sensor positioned at a distal portion of the guidewire and in electrical communication with the at least two conductive traces; and a connector positioned at a proximal portion of the guidewire and in electrical communication with the at least two conductive traces.

In some embodiments, the intraluminal sensing device further includes a third insulative coating surrounding an entire circumference of the core wire, over a top surface of the second insulative coating along at least the portion of the length of the core wire. In some embodiments, the third insulative coating covers portions of at least one of the conductive traces, the first insulative coating, or the core wire. In some embodiments, the at least two conductive traces include three conductive traces. In some embodiments, the conductive traces include a conductive ink. In some embodiments, the conductive ink is colloidal. In some embodiments, the conductive ink is sintered. In some embodiments, the conductive ink includes particles of at least one of gold, copper, silver, or aluminum. In some embodiments, at least two of the first insulative coating, the second insulative coating, or the third insulative coating include the same insulative material. In some embodiments, at least two of the first insulative coating, the second insulative coating, and the third insulative coating include different insulative materials. In some embodiments, each conductive trace includes a cross-sectional shape conforming to a curvature of the first insulative coating, and the at least two conductive traces, the at least two additional conductive traces, and the core wire are all mutually insulated from one another.

One general aspect includes an intraluminal sensing system. The system includes an intraluminal sensing guidewire and a processor circuit in communication with the intraluminal sensing guidewire. The processor circuit is configured to receive physiology data obtained by the sensor, process the physiology data, and output a graphical representation of the physiology data to a display in communication with the processing system. In some embodiments, the system further includes a patient interface module (PIM). Implementations may include hardware, a method or process, or computer software on a computer-accessible medium.

One general aspect includes a method for manufacturing an intraluminal sensing guidewire. The method includes providing a core wire; coating a first insulative material around an entire circumference of the core wire along at least a portion of a length of the core wire; coating a conductive ink over the first insulative material around the entire circumference of the core wire along at least the portion of the length of the core wire; coating a second insulative material over the conductive ink around the entire circumference of the core wire along at least the portion of the length of the core wire; removing material from at least two circumferential arcs of at least the second insulative material and the conductive ink, such that the conductive ink forms at least two longitudinal conductive traces along at least the portion of the length of the core wire, where the at least two longitudinal conductive traces are electrically isolated from each other and from the core wire; and coating a third insulative material over the second insulative material and the two circumferential arcs, around the entire circumference of the core wire along at least the portion of the length of the core wire.

In some embodiments, the at least two conductive traces include three conductive traces. In some embodiments, the method further includes sintering the conductive ink. In some embodiments, the conductive ink includes particles of gold, copper, silver, or aluminum. In some embodiments, at least two of the first insulative material, the second insulative material, or the third insulative material include the same insulative material. In some embodiments, at least two of the first insulative material, the second insulative material, and the third insulative material include different insulative materials.

One general aspect includes an intravascular sensing device including a guidewire configured to positioned with a blood vessel of a patient, including: a core wire; a first insulative coating covering at least a portion of a circumference of the core wire along at least a portion of a length of the core wire; at least two conductive traces including a sintered metallic ink, each conductive trace including a thickness and a width and extending longitudinally along at least the portion of the length of the core wire over an outer surface of the first insulative coating, where each conductive trace includes a cross-sectional shape conforming to a curvature of the first insulative coating, where the at least two conductive traces and the core wire are insulated from one another; a second insulative coating covering an outer surface of the at least two conductive traces along at least the portion of the length of the core wire; a third insulative coating surrounding an entire circumference of the core wire, over a top surface of the second insulative coating along at least the portion of the length of the core wire, where the third insulative coating covers portions of at least one of the conductive traces, the first insulative coating, or the core wire; at least one of a pressure sensor or a flow sensor positioned at a distal portion of the guidewire and in electrical communication with the at least two conductive traces; and a connector positioned at a proximal portion of the guidewire and in electrical communication with the at least two conductive traces.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the metal ink conductor assembly, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
**Figure 1** is a diagrammatic side view of an intravascular sensing system that includes an intravascular device comprising conductive members and conductive ribbons, according to aspects of the present disclosure.
**Figure 2** is a diagrammatic side view of another type of intravascular device, according to aspects of the present disclosure.
**Figure 3** is a perspective view of a multi-filar conductor bundle, according to aspects of the present disclosure.
**Figure 4** is a cross sectional view of at least a portion of an example proximal core wire assembly according to aspects of the present disclosure.
**Figure 5** is a cross sectional view of at least a portion of an example proximal core wire assembly according to aspects of the present disclosure.
**Figure 6** is a cross sectional view of at least a portion of an example proximal core wire assembly in accordance with at least one embodiment of the present disclosure.
**Figure 7** is a cross sectional view of at least a portion of an example proximal core wire assembly in accordance with at least one embodiment of the present disclosure.
**Figure 8** is a cross sectional view of at least a portion of an example proximal core wire assembly in accordance with at least one embodiment of the present disclosure.
**Figure 9** is a cross sectional view of at least a portion of an example proximal core wire assembly in accordance with at least one embodiment of the present disclosure.
**Figure 10** is a cross sectional view of at least a portion of an example proximal core wire assembly in accordance with at least one embodiment of the present disclosure.
**Figure 11** is a cross sectional view of at least a portion of an example proximal core wire assembly in accordance with at least one embodiment of the present disclosure.
**Figure 12** is a cross sectional view of at least a portion of an example proximal core wire assembly in accordance with at least one embodiment of the present disclosure.
**Figure 13** is a cross sectional view of at least a portion of an example proximal core wire assembly in accordance with at least one embodiment of the present disclosure.
**Figure 14** is a diagrammatic side view of an intravascular device comprising a reinforced multi-filar electrical conductor bundle and conductive traces, in accordance with at least one embodiment of the present disclosure.
**Figure 15** is a diagrammatic side view of the intravascular device of Figure 14, wherein additional coated longitudinal traces are embedded in an intravascular device in a distal core section.
**Figure 16** is a schematic diagram of a processor circuit, in accordance with at least one embodiment of the present disclosure.
**Figure 17** is a cross sectional view of at least a portion of an example core wire assembly in accordance with at least one embodiment of the present disclosure.

### DETAILED DESCRIPTION

Disclosed is a metal ink conductor assembly that provides a single-layer or multi-layer application of conductive metal ink traces which permit improved electrical/mechanical performance and eliminate manufacturing issues associated with conductive filars or ribbons. Manufacturing or assembly processes that require tensioning and coating of a multi-filar bundle, or one or more conductive ribbons, can benefit from the metal ink conductor assembly of the present disclosure.

Replacing embedded filars and/or flattened ribbon wires with coatable conductive traces (e.g., nano-metal ink traces) enables the use of conductive materials with lower tensile strengths and larger conductor cross-sections or surface areas, due to potential for greater utilization of available space within the insulation layer. This may enable lower resistance/impedance along the length of the electro-mechanical device. Because ribbons and/or multi-filar conductor bundles add stiffness and local torques to a guidewire or catheter, the straightness and torque response of the intraluminal device with coatable conductive traces may be improved as well, which may lead to a reduction in mechanical "whipping" responses when the device is manipulated within intravascular anatomy. Whipping may for example indicate a failure to rotate smoothly around a curve, leading the guidewire to jump or twitch inside the blood vessel.

Other manufacturing issues may also be alleviated by using the metal ink conductor assembly of the present disclosure. For example, the process of holding multiple filars and/or flattened ribbon wires in tension while laying them down and overcoating with an insulating layer is very difficult. Some current processes are only capable of being performed with two flattened ribbon wires or multi-filar conductor bundles per device. Conversely, the present disclosure enables the implementation of as many traces as desired along the length of device. Eliminating the elongation and necking of the filars and/or ribbon wires reduces the occurrence of blisters within the insulation layer. During device assembly, the twisting of filars, filar bundles, and flattened ribbon wires can make downstream laser ablation processes more difficult in terms of alignment. Furthermore, the processes used to create filars and flattened ribbon wires, and to embed them along the length of a device, are close to physical limits of the materials, due to the electrical/mechanical specifications of the electro-mechanical device. This creates design and sourcing limitations. The metal ink conductor assembly of the present disclosure reduces or eliminates these difficulties.

In the present disclosure, a conductive ink (e.g., nano-metal ink) is applied directly onto an insulating polymer layer (e.g., polyimide) as a liquid, allowing it to evenly coat and conform to the insulating surface. Each layer of ink is then sintered which turns the liquid into a substantially solid metal with material properties equivalent to bulk materials. After sintering, an additional insulation layer may be coated to a desired thickness. When the desired thickness is achieved, the nano-metal layer or layers can then be individuated to create traces with the desired cross-sectional area. After individuation, the traces are coated with additional insulating layers to electrically isolate them. This process may be repeated to create any desired number of layers and metallic traces.

Example devices incorporating a multi-filar conductor bundle and/or conductive ribbons include intraluminal medical guidewire devices as described for example in U.S. Patent No. 10,595,820 B2, U.S. Patent Publication Nos. 2014/0187874, 2016/0058977, and 2015/0273187, and in U.S. Provisional Patent Application No. 62/552,993 (filed August 31, 2017), each of which is hereby incorporated by reference in its entirety as though fully set forth herein.

These descriptions are provided for exemplary purposes only, and should not be considered to limit the scope of the metal ink conductor assembly. Certain features may be added, removed, or modified without departing from the spirit of the claimed subject matter.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. Further, while the embodiments of the present disclosure may be described with respect to a blood vessel, it will be understood that the devices, systems, and methods described herein may be configured for use in any suitable anatomical structure or body lumen including a blood vessel, blood vessel lumen, an esophagus, eustachian tube, urethra, fallopian tube, intestine, colon, and/or any other suitable anatomical structure or body lumen. In other embodiments, the devices, systems, and methods described herein may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood vessels, chambers or other parts of the heart, and/or other systems of the body. In addition to natural structures, the device 102 may be used to examine man-made structures such as, but without limitation, heart valves, stents, shunts, filters and other devices. For the sake of brevity, however, the numerous iterations of these combinations will not be described separately.

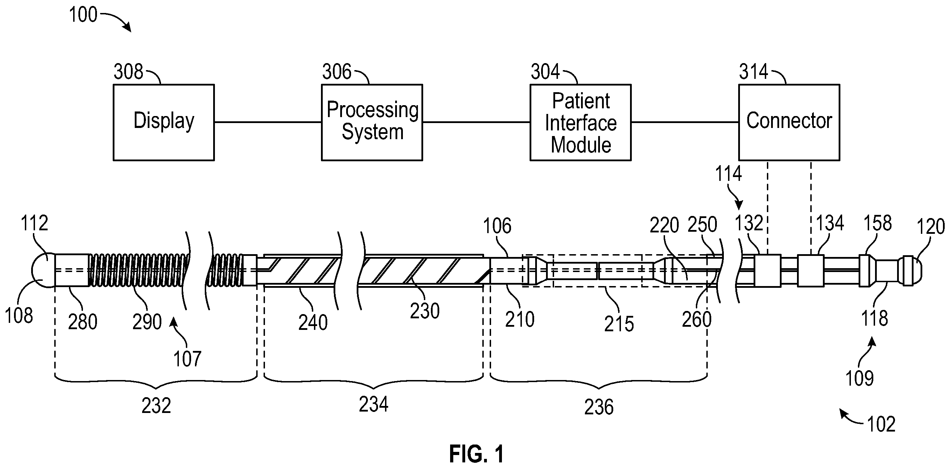

**Figure 1** is a diagrammatic side view of an intraluminal (e.g., intravascular) sensing system 100 that includes an intravascular device 102 comprising conductive members 230 (e.g., a multi-filar electrical conductor bundle) and conductive ribbons 260, according to aspects of the present disclosure. The intravascular device 102 can be an intravascular guidewire sized and shaped for positioning within a vessel of a patient. The intravascular device 102 includes a distal tip 108 and an electronic component 112. For example, the electronic component 112 can be a pressure sensor and/or flow sensor configured to measure a pressure of blood flow within the vessel of the patient, or another type of sensor including but not limited to a temperature or imaging sensor, or combination sensor measuring more than one property. For example, the flow data obtained by a flow sensor can be used to calculate physiological variables such as coronary flow reserve (CFR). The intravascular device 102 includes a flexible elongate member 106. The electronic component 112 is disposed at a distal portion 107 of the flexible elongate member 106. The electronic component 112 can be mounted at the distal portion 107 within a housing 280 in some embodiments. A flexible tip coil 290 extends distally from the housing 280 at the distal portion 107 of the flexible elongate member 106. A connection portion 114 located at a proximal end of the flexible elongate member 106 includes conductive portions 132, 134. In some embodiments, the conductive portions 132, 134 can be conductive ink that is printed and/or deposited around the connection portion 114 of the flexible elongate member 106. In some embodiments, the conductive portions 132, 134 are conductive, metallic bands or rings that are positioned around the flexible elongate member. A locking area is formed by a collar or locking section 118 and knob or retention section 120 are disposed at the proximal portion 109 of the flexible elongate member 106.

The intravascular device 102 in Figure 1 includes core wire comprising a distal core 210 and a proximal core 220. The distal core 210 and the proximal core 220 are metallic components forming part of the body of the intravascular device 102. For example, the distal core 210 and the proximal core 220 may be flexible metallic rods that provide structure for the flexible elongate member 106. The distal core 210 and/or the proximal core 220 can be made of a metal or metal alloy. For example, the distal core 210 and/or the proximal core 220 can be made of stainless steel, Nitinol, nickel-cobalt-chromium-molybdenum alloy (e.g., MP35N), and/or other suitable materials. In some embodiments, the distal core 210 and the proximal core 220 are made of the same material. In other embodiments, the distal core 210 and the proximal core 220 are made of different materials. The diameter of the distal core 210 and the proximal core 220 can vary along their respective lengths. A joint between the distal core 210 and proximal core 220 is surrounded and contained by a hypotube 215. The electronic component 112 can in some cases be positioned at a distal end of the distal core 210.

In some embodiments, the intravascular device 102 comprises a distal subassembly and a proximal subassembly that are electrically and mechanically joined together, which creates an electrical communication between the electronic component 112 and the conductive portions 132, 134. For example, flow data obtained by the electronic component 112 (in this example, electronic component 112 is a flow sensor) can be transmitted to the conductive portions 132, 134. In an exemplary embodiment, the flow sensor 112 is a single ultrasound transducer element. The transducer element emits ultrasound signals and receives echoes. The transducer element generates electrical signals representative of the echoes. The signal carrying filars carry this electrical signal from the sensor at the distal portion to the connector at the proximal portion. The processing system 306 processes the electrical signals to extract the flow velocity of the fluid.

Control signals from a processing system 306 (e.g., a processor circuit of the processing system 306) in communication with the intravascular device 102 can be transmitted to the electronic component 112 via a connector 314 that attached to the conductive portions 132, 134. The distal subassembly can include the distal core 210. The distal subassembly can also include the electronic component 112, the conductive members 230, and/or one or more layers of insulative polymer/plastic 240 surrounding the conductive members 230 and the core 210. For example, the polymer/plastic layer(s) can insulate and protect the conductive members of the multi-filar cable or conductor bundle 230. The proximal subassembly can include the proximal core 220. The proximal subassembly can also include one or more polymer layers 250 (hereinafter polymer layer 250) surrounding the proximal core 220 and/or conductive ribbons 260 embedded within the one or more insulative and/or protective polymer layer 250. In some embodiments, the proximal subassembly and the distal subassembly are separately manufactured. During the assembly process for the intravascular device 102, the proximal subassembly and the distal subassembly can be electrically and mechanically joined together. As used herein, flexible elongate member can refer to one or more components along the entire length of the intravascular device 102, one or more components of the proximal subassembly (e.g., including the proximal core 220, etc.), and/or one or more components the distal subassembly 410 (e.g., including the distal core 210, etc.). Accordingly, flexible elongate member may refer to the combined proximal and distal subassemblies described above. The joint between the proximal core 220 and distal core 210 is surrounded by the hypotube 215.

In various embodiments, the intravascular device 102 can include one, two, three, or more core wires extending along its length. For example, a single core wire can extend substantially along the entire length of the flexible elongate member 106. In such embodiments, a locking section 118 and a section 120 can be integrally formed at the proximal portion of the single core wire. The electronic component 112 can be secured at the distal portion of the single core wire. In other embodiments, such as the embodiment illustrated in Figure 1, the locking section 118 and the section 120 can be integrally formed at the proximal portion of the proximal core 220. The electronic component 112 can be secured at the distal portion of the distal core 210. The intravascular device 102 includes one or more conductive members 230 (e.g., a multi-filar conductor bundle or cable) in communication with the electronic component 112. For example, the conductive members 230 can be one or more electrical wires that are directly in communication with the electronic component 112. In some instances, the conductive members 230 are electrically and mechanically coupled to the electronic component 112 by, e.g., soldering. In some instances, the conductor bundle 230 comprises two or three electrical wires (e.g., a bifilar cable or a trifilar cable). An individual electrical wire can include a bare metallic conductor surrounded by one or more insulating layers. The conductive members 230 can extend along the length of the distal core 210. For example, at least a portion of the conductive members 230 can be spirally wrapped around the distal core 210, minimizing or eliminating whipping of the distal core within tortuous anatomy.

The intravascular device 102 includes one or more conductive ribbons 260 at the proximal portion of the flexible elongate member 106. The conductive ribbons 260 are embedded within polymer layer 250. The conductive ribbons 260 are directly in communication with the conductive portions 132 and/or 134. In some instances, a multi-filar conductor bundle 230 is electrically and mechanically coupled to the electronic component 112 by, e.g., soldering. In some instances, the conductive portions 132 and/or 134 comprise conductive ink (e.g., metallic nano-ink, such as copper, silver, gold, or aluminum nano-ink) that is deposited or printed directed over the conductive ribbons 260.

As described herein, electrical communication between the conductive members 230 and the conductive ribbons 260 can be established at the connection portion 114 of the flexible elongate member 106. By establishing electrical communication between the conductor bundle 230 and the conductive ribbons 260, the conductive portions 132, 134 can be in electrical communication with the electronic component 112.

In some embodiments represented by Figure 1, the intravascular device 102 includes a locking section 118 and a retention section 120. To form locking section 118, a machining process is used to remove polymer layer 250 and conductive ribbons 260 in locking section 118 and to shape proximal core 220 in locking section 118 to the desired shape. As shown in Figure 1, locking section 118 includes a reduced diameter while retention section 120 has a diameter substantially similar to that of proximal core 220 in the connection portion 114. In some instances, because the machining process removes conductive ribbons in locking section 118, proximal ends of the conductive ribbons 260 would be exposed to moisture and/or liquids, such as blood, saline solutions, disinfectants, and/or enzyme cleaner solutions, an insulation layer 158 is formed over the proximal end portion of the connection portion 114 to insulate the exposed conductive ribbons 260.

In some embodiments, a connector 314 provides electrical connectivity between the conductive portions 132, 134 and a patient interface monitor 304. The Patient Interface Monitor 304 may in some cases connect to a console or processing system 306, which includes or is in communication with a display 308.

The system 100 may be deployed in a catheterization laboratory having a control room. The processing system 306 may be located in the control room. Optionally, the processing system 306 may be located elsewhere, such as in the catheterization laboratory itself. The catheterization laboratory may include a sterile field while its associated control room may or may not be sterile depending on the procedure to be performed and/or on the health care facility. In some embodiments, device 102 may be controlled from a remote location such as the control room, such that an operator is not required to be in close proximity to the patient.

The intraluminal device 102, PIM 304, and display 308 may be communicatively coupled directly or indirectly to the processing system 306. These elements may be communicatively coupled to the medical processing system 306 via a wired connection such as a standard copper multi-filar conductor bundle 230. The processing system 306 may be communicatively coupled to one or more data networks, e.g., a TCP/IP-based local area network (LAN). In other embodiments, different protocols may be utilized such as Synchronous Optical Networking (SONET). In some cases, the processing system 306 may be communicatively coupled to a wide area network (WAN).

The PIM 304 transfers the received signals to the processing system 306 where the information is processed and displayed (e.g., as physiology data in graphical, symbolic, or alphanumeric form) on the display 308. The console or processing system 306 can include a processor and a memory. The processing system 306 may be operable to facilitate the features of the intravascular sensing system 100 described herein. For example, the processor can execute computer readable instructions stored on the non-transitory tangible computer readable medium.

The PIM 304 facilitates communication of signals between the processing system 306 and the intraluminal device 102. The PIM 304 can be communicatively positioned between the processing system 306 and the intraluminal device 102. In some embodiments, the PIM 304 performs preliminary processing of data prior to relaying the data to the processing system 306. In examples of such embodiments, the PIM 304 performs amplification, filtering, and/or aggregating of the data. In an embodiment, the PIM 304 also supplies high- and low-voltage DC power to support operation of the intraluminal device 102 via the conductive members 230.

A multi-filar cable or transmission line bundle 230 can include a plurality of conductors, including one, two, three, four, five, six, seven, or more conductors. In the example shown in Figure 1, the multi-filar conductor bundle 230 includes two straight portions 232 and 236, where the multi-filar conductor bundle 230 lies parallel to a longitudinal axis of the flexible elongate member 106, and a spiral portion 234, where the multi-filar conductor bundle 230 is wrapped around the exterior of the flexible elongate member 106 and then overcoated with an insulative and/or protective polymer 240. Communication, if any, along the multi-filar conductor bundle 230 may be through numerous methods or protocols, including serial, parallel, and otherwise, wherein one or more filars of the bundle 230 carry signals. One or more filars of the multi-filar conductor bundle 230 may also carry direct current (DC) power, alternating current (AC) power, or serve as a ground connection.

The display or monitor 308 may be a display device such as a computer monitor or other type of screen. The display or monitor 308 may be used to display selectable prompts, instructions, and visualizations of imaging data to a user. In some embodiments, the display 308 may be used to provide a procedure-specific workflow to a user to complete an intraluminal imaging procedure.

Before continuing, it should be noted that the examples described above are provided for purposes of illustration, and are not intended to be limiting. Other devices and/or device configurations may be utilized to carry out the operations described herein.

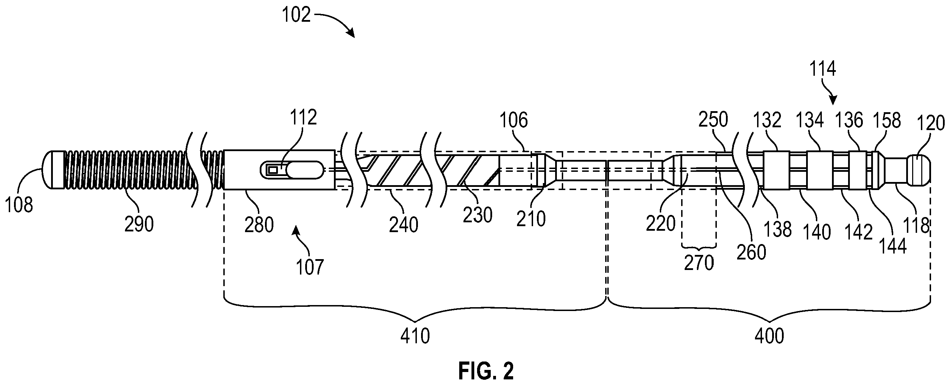

Figure 2 is a side view of another type of intravascular device 102, according to aspects of the present disclosure. The intravascular device 102 can be an intravascular guidewire sized and shaped for positioning within a vessel of a patient. The intravascular device 102 can include an electronic component 112. For example, the electronic component 112 can be a pressure sensor configured to measure a pressure of blood flow within the vessel of the patient, or another type of sensor. Pressure data obtained by a pressure sensor may for example be used to calculate a physiological pressure ratio (e.g., FFR, iFR, Pd/Pa, or any other suitable pressure ratio). However, the device 102 may be used in any suitable anatomical structure or body lumen including a blood vessel, blood vessel lumen, an esophagus, eustachian tube, urethra, fallopian tube, intestine, colon, and/or any other suitable anatomical structure or body lumen.

The intravascular device 102 includes a flexible elongate member 106, such as a guidewire. The electronic component 112 is disposed at the distal portion 107 of the flexible elongate member 106. The electronic component 112 can be mounted at the distal portion 107 within a housing 280 in some embodiments. A flexible tip coil 290 extends between the housing 280 and the distal end 108. The connection portion 114 is disposed at the proximal portion of the flexible elongate member 106. The connection portion includes the conductive portions 132, 134, 136. In some embodiments, the conductive portions 132, 134, 136 can be conductive ink that is printed and/or deposited around the flexible elongate member. In some embodiments, the conductive portions 132, 134, 136 are conductive, metallic rings or bands that are positioned around the flexible elongate member. The locking section 118 and retention section 120 are disposed at the proximal portion of the flexible elongate member 106.

In some embodiments, the intravascular device 102 comprises a distal subassembly 410 and a proximal subassembly 400 that are electrically and mechanically coupled, which provides for electrical communication between the electronic component 112 and the conductive portions 132, 134, 136. For example, pressure data obtained by the electronic component 112 (in this example, electronic component 112 is a pressure sensor) can be transmitted to the conductive portions 132, 134, 136. Control signals from a processing system in communication with the intravascular device 102 can be transmitted to the electronic component 112 via the conductive portions 132, 134, 136. The distal subassembly 410 can include the distal core 210. The distal subassembly 410 can also include the electronic component 112, the conductive members 230, and/or one or more layers of polymer/plastic 240 surrounding the conductive members 230 and the core 210. For example, the polymer/plastic layer(s) can protect the conductive members 230. The proximal subassembly 400 can include the proximal core 220. The proximal subassembly 400 can also include one or more polymer layers 250 surrounding the proximal core 220 and/or conductive ribbons 260 embedded within the one or more polymer layers 250. In some embodiments, the proximal subassembly 400 and the distal subassembly 410 can be separately manufactured. During the assembly process for the intravascular device 102, the proximal subassembly 400 and the distal subassembly 410 can be electrically and mechanically joined together, and the joint can be enclosed in a hypotube 215. As used herein, flexible elongate member can refer to one or more components along the entire length of the intravascular device 102, one or more components of the proximal subassembly 400 (e.g., including the proximal core 220, etc.), and/or one or more components the distal subassembly 410 (e.g., including the distal core 210, etc.).

In various embodiments, the intravascular device 102 can include one, two, three, or more core wires extending along its length. For example, a single core wire can extend substantially along the entire length of the flexible elongate member 106. In such embodiments, a locking section 118 and a retention section 120 can be integrally formed at the proximal portion of the single core wire. The electronic component 112 can be secured at the distal portion of the single core wire. In other embodiments, such as the embodiment illustrated in Figure 2, the locking section 118 and the retention section 120 can be integrally formed at the proximal portion of the proximal core 220. The electronic component 112 can be secured at the distal portion of the distal core 210. The intravascular device 102 includes one or more conductive members 230 in communication with the electronic component 112. For example, the conductive members 230 can be one or more electrical wires that are directly in communication with the electronic component 112.

The intravascular device 102 includes one or more conductive ribbons 260 at the proximal portion of the flexible elongate member 106. The conductive ribbons 260 are embedded within polymer layer(s) 250. The conductive ribbons 260 are directly in communication with the conductive portions 132, 134, and/or 136. In some instances, the conductive members 230 are electrically and mechanically coupled to the electronic component 112 by, e.g., soldering, welding, terminals, clamps, conductive adhesive, or other appropriate methods. In some instances, the conductive portions 132, 134, and/or 136 comprise conductive ink (e.g., metallic nano-ink, such as silver or gold nano-ink) that is deposited or printed directed over the conductive ribbons 260.

As described herein, electrical communication between the conductive members 230 and the conductive ribbons 260 can be established at the connection region 270 of the flexible elongate member 106. By establishing electrical communication between the conductive members 230 and the conductive ribbons 260, the conductive portions 132, 134, 136 can be in electrically communication with the electronic component 112.

In some instances, the machining process that forms locking section 118 may remove of the conductive ribbons 260, proximal ends of the conductive ribbons 260 would be exposed to moisture and/or liquids, such as blood, saline solutions, disinfectants, and/or enzyme cleaner solutions. In these cases, an insulation layer 158 can be formed over the proximal end portion of the connection portion 114 to insulate the exposed conductive ribbons 260.

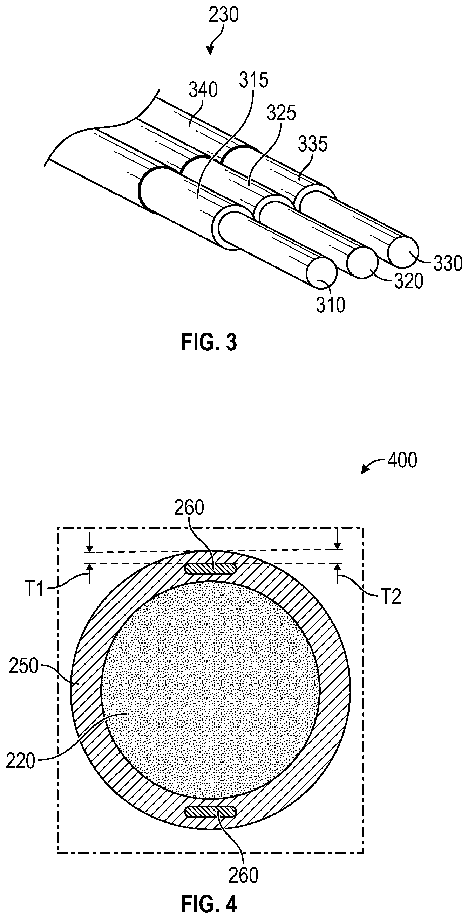

Figure 3 is a perspective view of a multi-filar conductor bundle 230 in accordance with aspects of the present disclosure. In the example shown in Figure 3, the multi-filar conductor bundle 230 is a trifilar that includes three conductors 310, 320, and 330, surrounded by insulating sheaths 315, 325, and 335, respectively. The insulating sheaths 315, 325, 335, may, for example, comprise polyimide, and may or may not be covered with an additional overcoating 340, such as nylon or polyurethane, that joins the separate insulating sheaths 315, 325, and 335 together, such that the filars 310, 320, and 330 form a single joined conductor bundle 230. The additional overcoating 340 may for example be applied through dip coating, although other methods may be used instead or in addition. For example, the individual insulating sheaths 315, 325, 335 may be attached to one another using an adhesive, by welding, or any other suitable approach. In some embodiments, the insulating sheaths 315, 325, 335 are formed or extruded over the conductors in a single processing step. In a conventional trifilar, all three conductors may be of the same or similar diameter, and may be made of pure copper, or of a copper alloy such as BeCu. Although the multi-filar conductor bundle 230 is shown here with the conductors arranged side-by-side in a planar fashion, a person of ordinary skill in the art will appreciate that other arrangements may be employed instead or in addition.

During the manufacturing process for an intravascular device, the filars may be wrapped or pulled along the length of the device, from one end of the device to the other end. Because pure copper and many copper alloys have a low tensile and yield strength, the mechanical strength of electrical conductor materials is typically very low. The manufacturing process for the intravascular device may involve wrapping or stretching of the multi-filar conductor bundle, which may involve tensioning. Unfortunately, this tensioning can cause the conductive filars to plasticly deform, which can result in severe and undesirable elongation and/or necking, e.g., up to 100% elongation in some current processes, along with blistering of the insulation around the necking site. The associated narrowing of the conductor affects both the mechanical strength and the maximum electrical current capacity of the filar, which can lead to manufacturing defects. It may therefore be desirable to replace the filars with other types of conductors, and/or replace the manufacturing process with a process that does not require pulling, wrapping, or tensioning of the conductors.

**Figure 4** is a cross sectional view of at least a portion of an example proximal core wire assembly 400 according to aspects of the present disclosure. Visible are the proximal core wire 220, two conductive ribbons 260, and a layer of insulator 250. In some embodiments, conductive ribbons 260 can also be used in place of multi-filar conductor bundles 230 in the distal core wire assembly 410 in a similar manner to what is shown here in Figure 4. The example shown here is idealized, with all components aligned in an intended manner, with no manufacturing defects. However, even in this idealized case, the thickness T1 of the insulator 250 over the left and right edges of the conductive ribbon 260 is smaller than the thickness T2 of the insulator 250 over the center of the conductive ribbon 260. This occurs because the ribbon 260 comes off a spool in a flat shape, and does not conform to the curvature of the core wire 220. Similarly, the thickness of the insulator 250 under the left and right edges of the conductive ribbon 260 is greater than the thickness of the insulator 250 under the center of the conductive ribbon 260. The inherent stiffness of the flat conductive ribbons 260 also increases the stiffness of the distal core wire assembly 410, and increases the chance of fatigue-related weakening of the insulation or other durability problems, especially where the insulation 250 is thinnest. Another issue with using flat conductive ribbons 260 in this manner is that tensional variations can, in some cases, also effect the straightness of the proximal core wire section beyond what is considered acceptable for clinical use.

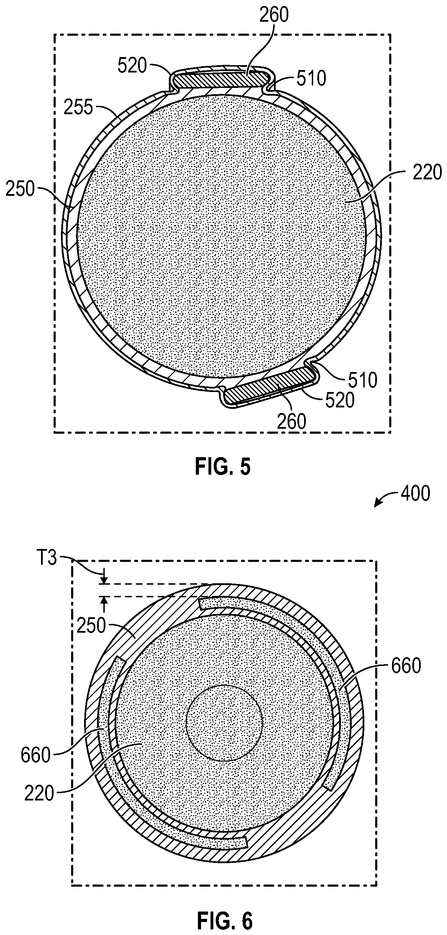

**Figure 5** is a cross sectional view of at least a portion of an example proximal core wire assembly 400 according to aspects of the present disclosure. This view is a photograph that shows a possible configuration of the elements of Figure 4, in a non-idealized, manufactured product. Visible are the core wire 220, conductive ribbons 260, inner insulative coating 250, and outer insulative coating 255. As can be seen in the image, alignment or titling of the flat ribbons 260 against the round core wire 220 can create gaps 510 and thin spots 520 in the insulative coating 250. It is noted that the ribbons 260 are substantially flat because they have been unwound from a spool during the assembly process. Due to the small cross-section and relatively rigid nature of the ribbons 260, the ribbons 260 do not conform to the curvature of the core wire.

**Figure 6** is a cross sectional view of at least a portion of an example proximal core wire assembly 400 in accordance with at least one embodiment of the present disclosure. In this configuration, conformal metallic traces 660 are used as conductors instead of ribbon conductors, which are shown in the embodiment of Figure 1, for example. The metallic traces 660 are made from a coated, conductive ink, such as a nano-metallic ink. Because the traces 660 follow the curve of the core wire 220, the insulator 250 maintains a constant thickness T3 above the traces 660, regardless of position. This permits the conductors 660 to be wider and have a larger cross-sectional area (and therefore greater current carrying capacity) than the ribbons 260, without decreasing the thickness of the insulation 250 at the edges, and without increasing, or significantly increasing, the thickness of insulation 250 used to fully cover and insulate the traces 660. Furthermore, because the traces 660 conform to the shape of the core wire 220, they do not add as much stiffness to the proximal core wire assembly 400, and therefore improve the durability and fatigue resistance of the insulator 250. Another important aspect of the nano-ink trace 660 is that it allows a much wider trace, and therefore it may allow for an overall thinner trace that supplies same electrical qualities as the flattened ribbons. This can be an important feature when considering proximal core diameter requirements. A larger core diameter, with thinner traces stacked on top of it, can provide better control of the wire by the operator by providing better push and torqueability.

Because the traces 660 are coated or deposited rather than wrapped or tensioned, they can for example be made of mechanically weak conductor materials such as pure metallic copper, without the risk of elongation or necking during the manufacturing process. Additionally, the deposited traces 660 conform to the shape or curvature of the wire, which can serve to reduce the cross-sectional size or profile of the device 102. Conductive nano-metal inks may be employed for this production process. The conductive nano-metal ink can be applied in many ways, including but not limited to inkjet printing, aerosol-jet printing, felt/foam pad applicator, or dip coating. In some embodiments, the material is cured or dried, and the traces are formed from the cured or dried material. In some embodiments, the coated material is sintered, e.g., heated until the small metal particles in the coated ink melt together. Sintering can be accomplished with an oven, a laser, a torch, or by other means. Once cured, sintered, or otherwise turned into a solid metal, the coated metal layer or layers can be individuated via laser ablation, mechanical cutting/skiving or any other methods capable of removing metal and/or polymer material in a precise manner. This process can be implemented on continuous reel to reel type combination coater/oven equipment. In one example, ink application is performed with an ink-impregnated pad made of felt or foam, and individuation is performed by mechanical cutting. In another example, ink application is performed with an ink-impregnated felt or foam pad, and individuation is performed via laser ablation. In still another example, the ink application is performed by dip coating. This metal ink conductor assembly could be applied to any product that employs electrical conductors embedded into composite subassemblies.

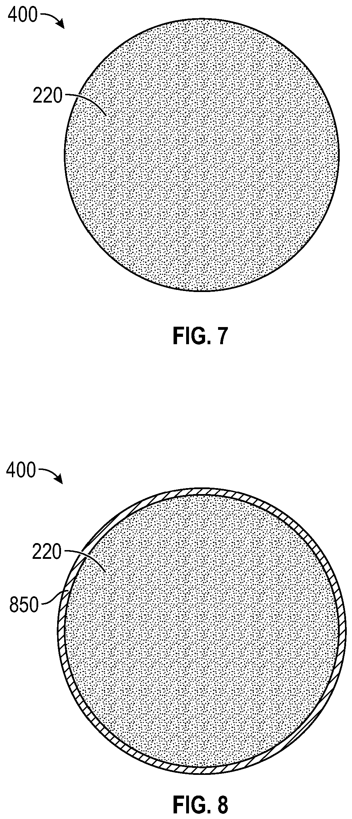

Figures 7-12 depict processing, assembly, or manufacturing steps that may be employed to create the conformal conductive traces 660 that are shown for example in Figure 6.

**Figure 7** is a cross sectional view of at least a portion of an example proximal core wire assembly 400 in accordance with at least one embodiment of the present disclosure. An example manufacturing process begins with a core wire 220, shown here in cross section. The core wire 220 may for example be made of stainless steel, although other materials may be used instead or in addition, including Nitinol or MP35N.

**Figure 8** is a cross sectional view of at least a portion of the example proximal core wire assembly 400 in accordance with at least one embodiment of the present disclosure. In a first step of the example manufacturing process, a polymer coating 850 is applied to at least a portion of the core wire 220. The polymer coating 850 may for example comprise polyimide, polyamide, or other polymers depending on the implementation. The polymer coating 850 may be applied for example by jetting, spray-coating, dip coating, coating by a soaked pad of felt or foam, or any other suitable technique.

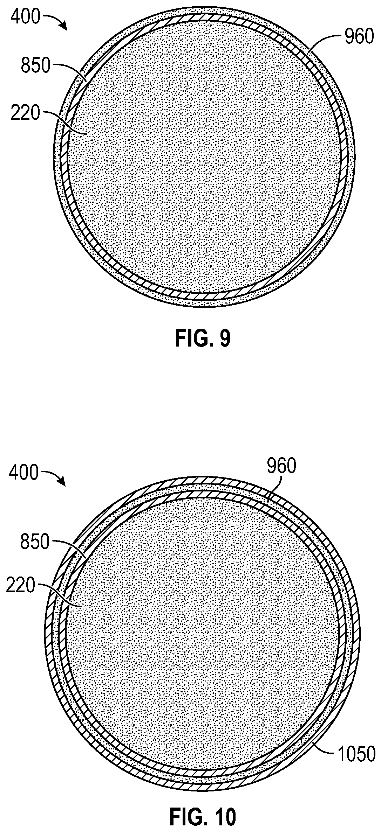

**Figure 9** is a cross sectional view of at least a portion of the example proximal core wire assembly 400 in accordance with at least one embodiment of the present disclosure. In a second step of the example manufacturing process, a conductive ink layer 960 is coated on the exterior of at least a portion of the polymer coating 850. The conductive ink 960 may, for example, comprise microscopic or nanoscopic particles of a conductive polymer (e.g., polyacetylene, polypyrrole, or polyaniline) or a metal (e.g., copper, silver, gold, or aluminum), suspended or solvated in a liquid carrier material that evaporates after the coating step, either spontaneously or with the help of a drying oven or other heating or drying equipment, leaving behind a colloidal film of conductive particles. The conductive nano-metal ink can be applied by a variety of different methods, including but not limited to inkjet printing, aerosol-jet printing, felt/foam pad applicator, or dip coating. In some embodiments, once the carrier material has evaporated and the conductive material has dried and/or cured, the deposited conductor material 960 can be employed in its coated (colloidal) state. In some embodiments, electrical conductivity and mechanical strength of the conductive traces can be further improved through sintering, which may involve heating all or portions of the material until the small conductive particles in the coated ink melt together into a substantially continuous solid, with properties resembling those of a bulk material. Sintering can be accomplished with an oven, a laser, a torch, or by any other suitable technique.

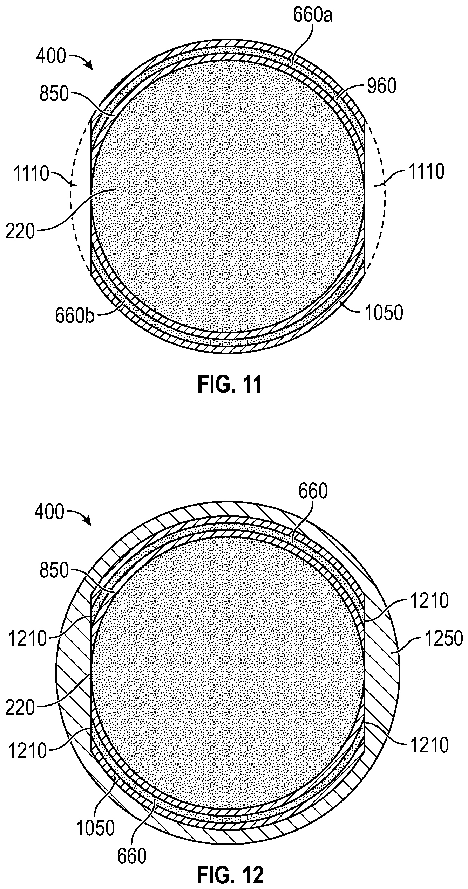

In some embodiments, the conductive ink or material may be applied only to portions of the circumference, and not around the entire circumference. For example, longitudinal strips may be printed or coated on so that only portions of the circumference of the polymer coating are covered with the conductive ink. In these embodiments, individual traces are applied to the insulated core, using, e.g., inkjet printing, continuous reel-to-reel process, etc. The individual traces can be applied one at a time or can be simultaneously applied. These embodiments may or may not employ the manufacturing steps depicted in Figures 10 and 11.

**Figure 10** is a cross sectional view of at least a portion of the example proximal core wire assembly 400 in accordance with at least one embodiment of the present disclosure. In a third step of the example manufacturing process, a second polymer coating 1050 is applied to at least a portion of the conductive coating 960. The polymer coating 1050 may be of the same or a different material than the first polymer coating 850, and may be applied by the same or a different coating process. The polymer coating 1050 advantageously protect the edges of the conductive traces created during the cutting or skiving process (as described with reference to, e.g., Fig. 11), which may cause the metal/conductive material to lift or buckle if there is no coating over the metal/conductive material.

**Figure 11** is a cross sectional view of at least a portion of the example proximal core wire assembly 400 in accordance with at least one embodiment of the present disclosure. In a fourth step of the example manufacturing process, two or more cuts 1110 are made to at least a portion of the proximal core wire assembly such that portions of at least the outer polymer coating 1050 and coated conductive layer 960 are removed. Depending on the implementation, portions of the inner polymer coating 850 may also be removed, and in some cases the cuts 1110 may also remove a small amount of material from the core wire 220, although it may be beneficial to prevent or minimize this occurrence. Once the cuts 1110 are complete, the coated conductive layer 960 are divided into singulated regions, which form longitudinal traces 660a and 660b that conform to the curvature of the core wire 220 and/or the curvature of the inner polymer coating 850, as shown for example in Figure 6. In an example, the longitudinal traces 660a and 660b each have arcuate profiles that form portions of a cylinder that is concentric with the core wire 220 and/or the first insulative coating 850.

It is noted that the cuts 1110 may, in some instances, be made before the outer polymer coating 1050 is deposited. However, this may tend to result in rougher edges and less regular depth, whereas cutting or skiving the metal layer 960 along with the outer polymer coating may tend to result in cleaner cuts.

**Figure 12** is a cross sectional view of at least a portion of the example proximal core wire assembly 400 in accordance with at least one embodiment of the present disclosure. In a fifth step of the example manufacturing process, a third polymer coating 1250 is applied to the outer surface of the singulated proximal core wire assembly 400. With some coating processes, because of surface tension, this outer polymer coating 1250 may be thicker in regions where the singulated proximal core wire assembly 400 has a lower profile, and thinner in regions where the singulated proximal core wire assembly 400 has a larger profile. As a result, the cross section of the proximal core wire assembly 400, including the outer polymer coating 1250 may tend to be circular, or approximately circular. However, other cross-sectional shapes are also contemplated, including elliptical, oblong, oval, rectangular, triangular, or any other suitable cross-sectional shape. The outer polymer coating 1250 insulates the cut edges 1210 of the longitudinal traces 660, thus ensuring that they are electrically insulated from contact with one another, with the core wire 220, and with conductive objects external to the proximal core wire assembly 400.

In some embodiments, longitudinal traces 660 formed with conductive ink can be used in place of multi-filar conductor bundles 230 in the distal core wire assembly 410, instead of or in addition to replacing conductive metallic ribbons 260 in the proximal core wire assembly 400. In some embodiments, the outer polymer coating 1250 may be applied directly over the conductive traces 660, and the polymer coating 1050 may not be applied at all. In some embodiments, additional coatings may be applied over the outer polymer coating 1250 to, for example, reduce friction, improve lubricity, or alter the wetting properties of the device.

Depending on the implementation, the structure shown in Figure 12 can be formed around a proximal core wire, a distal core wire, or a core wire formed of a proximal core wire and a distal core wire.

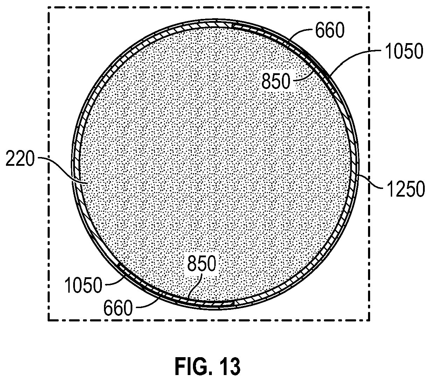

**Figure 13** is a cross sectional view of at least a portion of an example proximal core wire assembly 400 in accordance with at least one embodiment of the present disclosure. This view is a photograph that shows a possible configuration of the elements of Figure 12, in a non-idealized, manufactured product. Visible are the core wire 220, conductive traces 660, and insulative coatings 850, 1050, and 1250. The conductive traces 660 are completely surrounded by the insulative coatings 850 and/or 1050 such that the conductive traces 660 are electrically insulated from the core wire 220. The core wire 220 is also completely surrounded by the insulative coatings 850, 1050, and 1250. For example, the outer surface of the core 220 can be in contact with the insulative coatings 850, 1050, and /or 1250. As can be seen in the image, the insulative coating 1250 is substantially free of the voids and thin spots visible in Figure 5.

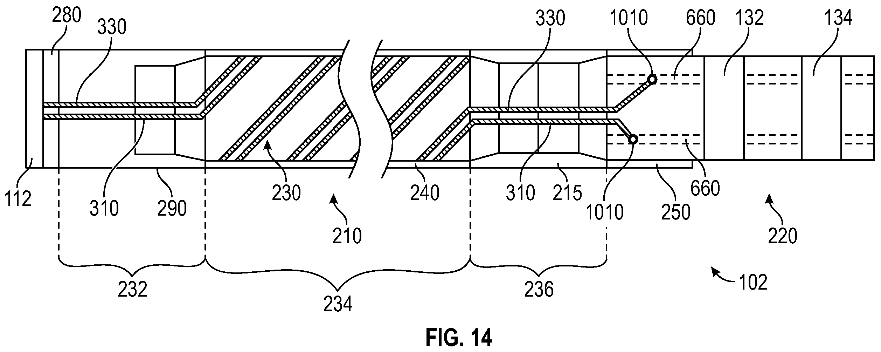

**Figure 14** is a diagrammatic side view of an intravascular device 102 comprising a reinforced multi-filar electrical conductor bundle 230 and coated conductive traces 660, in accordance with at least one embodiment of the present disclosure. Visible are the proximal core wire 220 and distal core wire 210, joined by a hypotube 215. At the distal end of the distal core wire is a coil 290 that terminates with an electronic device 112 that may be fully or partially enclosed within a housing 280. Also visible is a reinforced multi-filar conductor bundle 230 that includes conductive filars 310 and 330. In some embodiments, the electronic device 112 may be located at a proximal end of the coil 290 instead of being at a distal end of the coil 290. In such embodiments, the conductive filars 310 and 330 would not extend into the coil 290 and rather terminate proximal of the coil 290, at the electronic device 112. In some embodiments, one electronic device 112 can be at the distal end of the intravascular device 102, and a different electronic device 112 can be spaced from the distal end.

The conductive filars 310 and 330 connect the electronic component 112 with electrical contacts 1010 formed on the conformal, coated longitudinal traces 660 that make electrical contact with the conductive regions 132 and 134. The reinforced multi-filar conductor bundle 230 includes a straight region 232 that passes through or along the coil 290. The reinforced multi-filar conductor bundle 230 also includes a spiral region 234 that wraps around the distal core wire 210 and is overcoated with an insulative or protective polymer coating 240. The reinforced multi-filar conductor bundle 230 additionally includes a straight region 236 that passes through the hypotube 215. Between the hypotube 215 and the electrical contacts 1010, the conductive filars are overcoated with a polymer coating 250. Placement of the multi-filar conductor bundle in these regions 232, 234, and 236 may involve tensioning of the multi-filar conductor bundle, with attendant risk of unwanted elongation and/or necking of the conductors 230.

The device 102 can include any suitable quantity of conductors in the bundle 230, include two, three, four, five, or more. The device 102 can include any suitable quantity of conductive traces 660, include two, three, four, five, or more. In some embodiments, the device 102 includes the same quantity of conductors in the bundle 230 as conductive traces 660. In some embodiments, the quantity of conductors in the bundle 230 is greater than or less than the quantity of conductive traces 660. In that regard, in some embodiments, the bundle 230 includes one more conductor in the bundle 230 as the quantity of conductive traces 660. The one additional conductor in the bundle 230 can be electrically and mechanically terminated at the core wire 220 to provide an electrical ground. The device 102 can include any suitable number of conductive regions 132 and 134, include two, three, four, five or more. In some embodiments, the device 102 includes the same quantity of conductive traces 660 as conductive regions 132 and 134.

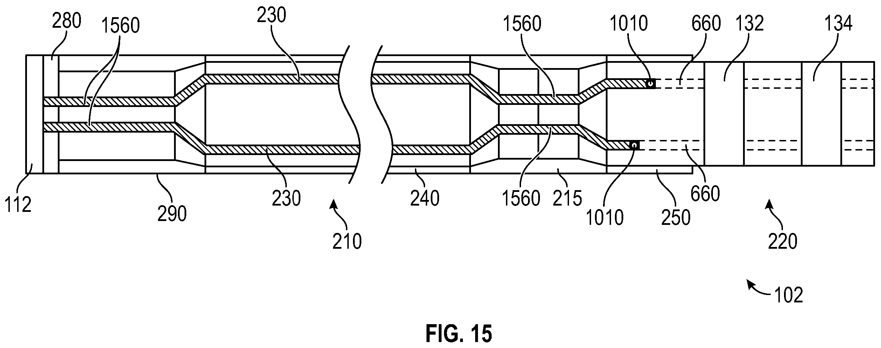

**Figure 15** is a diagrammatic side view of the intravascular device 102 of Figure 14, wherein the multi-filar electrical conductor bundle 230 has been replaced with additional coated longitudinal traces 1560. The coated longitudinal traces 1560 connect the electronic component 112 with electrical contacts 1010 formed on the coated longitudinal conductive traces 660 that make electrical contact with the conductive regions 132 and 134. The coated longitudinal traces 1560 include a region that passes through the coil 290 and then follows the distal core wire 210, and is overcoated with an insulative or protective polymer coating 240. The coated longitudinal traces 1560 additionally include a region that passes through the hypotube 215. Between the hypotube 215 and the electrical contacts 1010, the conductive traces 1560 are overcoated with a polymer coating 250.

Placement of the coated conductive traces 1560 in these regions does not require tensioning, with attendant risk of unwanted elongation and/or necking, and may therefore represent an improvement over the use of multi-filar conductor bundles.

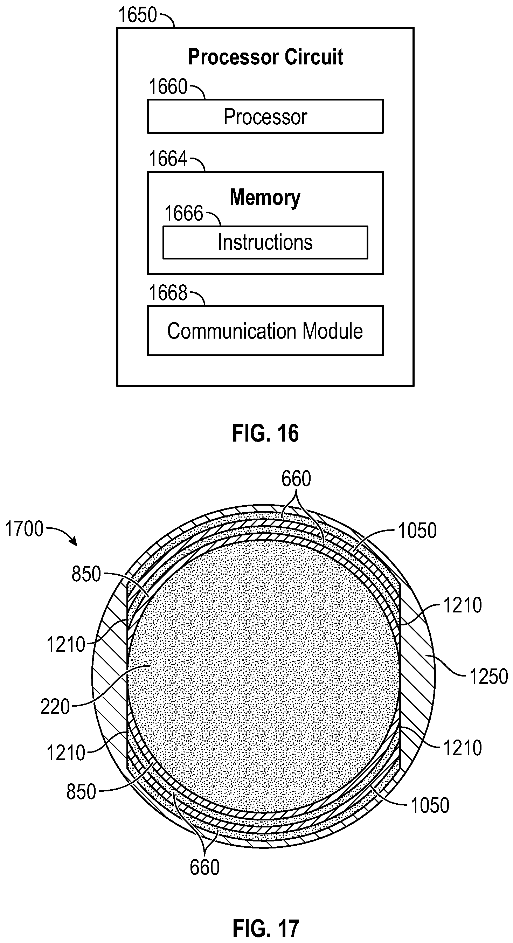

**Figure 16** is a schematic diagram of a processor circuit 1650, according to at least one embodiment of the present disclosure. The processor circuit 1650 may be implemented in the intravascular sensing system 100 (e.g., the processing system 306) or other devices or workstations (e.g., third-party workstations, network routers, etc.), or on a cloud processor or other remote processing unit, as necessary to implement the method. As shown, the processor circuit 1650 may include a processor 1660, a memory 1664, and a communication module 1668. These elements may be in direct or indirect communication with each other, for example via one or more buses.

The processor 1660 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1660 may also comprise another hardware device, a firmware device, or any combination thereof configured to perform the operations described herein. The processor 1660 may also be implemented as a combination of computing devices, e.g., a combination of a DSP and a microprocessor, a plurality of microprocessors, one or more microprocessors in conjunction with a DSP core, or any other such configuration.

The memory 1664 may include a cache memory (e.g., a cache memory of the processor 1660), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1664 includes a non-transitory computer-readable medium. The memory 1664 may store instructions 1666. The instructions 1666 may include instructions that, when executed by the processor 1660, cause the processor 1660 to perform the operations described herein. Instructions 1666 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1668 can include any electronic circuitry and/or logic circuitry to facilitate direct or indirect communication of data between the processor circuit 1650, and other processors or devices. In that regard, the communication module 1668 can be an input/output (I/O) device. In some instances, the communication module 1668 facilitates direct or indirect communication between various elements of the processor circuit 1650 and/or the intravascular measurement system 100. The communication module 1668 may communicate within the processor circuit 1650 through numerous methods or protocols. Serial communication protocols may include but are not limited to US SPI, I²C, RS-232, RS-485, CAN, Ethernet, ARINC 429, MODBUS, MIL-STD-1553, or any other suitable method or protocol. Parallel protocols include but are not limited to ISA, ATA, SCSI, PCI, IEEE-488, IEEE-1284, and other suitable protocols. Where appropriate, serial and parallel communications may be bridged by a UART, USART, or another appropriate subsystem.

External communication (including but not limited to software updates, firmware updates, preset sharing between the processor and central server, or readings from the intraluminal device) may be accomplished using any suitable wireless or wired communication technology, such as a cable interface such as a USB, micro USB, Lightning, or FireWire interface, Bluetooth, Wi-Fi, ZigBee, Li-Fi, or cellular data connections such as 2G/GSM, 3G/UMTS, 4G/LTE/WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

**Figure 17** is a cross sectional view of at least a portion of an example core wire assembly 1700 in accordance with at least one embodiment of the present disclosure. Visible is a core wire 220, which may be a proximal core wire, a distal core wire, or a core wire comprising a proximal and a distal core wire. Also visible are two layers of traces 660, along with a first insulative coating 850, a second insulative coating 1050, and a third insulative coating 1250. Depending on the implementation, the insulative coatings 850, 1050, and 1250 may all be the same material, may all be different materials, or any two of the three insulative coatings may be the same material.

Accordingly, it can be seen that the metal ink conductor assembly advantageously reduces or eliminates the need for tensioning of multi-filar conductor bundles and/or conductive ribbons that may be used in the manufacture of small electronic devices such as intravascular medical catheters and guidewires. This may tend to eliminate the risk of elongation and/or necking that can compromise the mechanical and electrical properties of a conductor, as well as reducing the risk of gaps or thin spots in the outer insulative coating. A number of variations are possible on the examples and embodiments described above. For example, the conductive ink could be coated in the form of individual traces, rather than a uniform coating that is later individuated by cutting.

The metal ink conductor assembly could be applied to any product that involves electrical conductors to be embedded into composite subassemblies. The present disclosure permits conductors with similar cross-sectional area to flat conductive ribbons. However, unlike conductive ribbons, these conductors can conform to the curvature of the core wire. The present disclosure also creates detectable material transition layers in the areas where individuation occurs.

The logical operations making up the embodiments of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use electrical and electronic devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the metal ink conductor assembly. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the metal ink conductor assembly as defined in the claims. Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

### Clauses

Clause 1. An intraluminal sensing device, comprising:
   a guidewire configured to be positioned within a body lumen of a patient, wherein the guidewire comprises:
   a core wire;
   a first insulative coating covering at least a portion of a circumference of the core wire along at least a portion of a length of the core wire;
   at least two conductive traces, each comprising a thickness and a width and extending longitudinally along at least the portion of the length of the core wire over an outer surface of the first insulative coating, wherein each conductive trace comprises a cross-sectional shape conforming to a curvature of the first insulative coating, wherein the at least two conductive traces and the core wire are insulated from one another; and
   a second insulative coating covering an outer surface of the at least two conductive traces along at least the portion of the length of the core wire;
   a sensor configured to obtain physiological data while positioned within the body lumen, wherein the sensor is positioned at a distal portion of the guidewire and in electrical communication with the at least two conductive traces; and
   a connector positioned at a proximal portion of the guidewire and in electrical communication with the at least two conductive traces.
Clause 2. The intraluminal sensing device of clause 1, further comprising a third insulative coating surrounding an entire circumference of the core wire, over a top surface of the second insulative coating along at least the portion of the length of the core wire.
Clause 3. The intraluminal sensing device of clause 2, wherein the third insulative coating covers portions of at least one of the conductive traces, the first insulative coating, or the core wire.
Clause 4. The intraluminal sensing device of clause 2, wherein the at least two conductive traces comprise three conductive traces.
Clause 5. The intraluminal sensing device of clause 2, wherein the conductive traces comprise a conductive ink.
Clause 6. The intraluminal sensing device of clause 5, wherein the conductive ink is colloidal.
Clause 7. The intraluminal sensing device of clause 5, wherein the conductive ink is sintered.
Clause 8. The intraluminal sensing device of clause 5, wherein the conductive ink comprises particles of at least one of gold, copper, silver, or aluminum.
Clause 9. The intraluminal sensing device of clause 2, wherein at least two of the first insulative coating, the second insulative coating, or the third insulative coating comprise the same insulative material.
Clause 10. The intraluminal sensing device of clause 2, wherein at least two of the first insulative coating, the second insulative coating, and the third insulative coating comprise different insulative materials.
Clause 11. The intraluminal sensing device of clause 2, further comprising at least two additional conductive traces, each having a thickness and a width, each running longitudinally along at least the portion of the length of the core wire over an outer surface of the second insulative coating,
   wherein each conductive trace comprises a cross-sectional shape conforming to a curvature of the first insulative coating, and
   wherein the at least two conductive traces, the at least two additional conductive traces, and the core wire are all mutually insulated from one another.
Clause 12. An intraluminal sensing system, comprising:
   the intraluminal sensing guidewire of clause 1; and
   a processor circuit in communication with the intraluminal sensing guidewire, wherein the processor circuit is configured to receive physiology data obtained by the sensor, process the physiology data, and output a graphical representation of the physiology data to a display in communication with the processing system.
Clause 13. The system of clause 12, further comprising a patient interface module (PIM).
Clause 14. A method for manufacturing an intraluminal sensing guidewire, comprising:
   providing a core wire;
   coating a first insulative material around an entire circumference of the core wire along at least a portion of a length of the core wire;
   coating a conductive ink over the first insulative material around the entire circumference of the core wire along at least the portion of the length of the core wire;
   coating a second insulative material over the conductive ink around the entire circumference of the core wire along at least the portion of the length of the core wire;
   removing material from at least two circumferential arcs of at least the second insulative material and the conductive ink, such that the conductive ink forms at least two longitudinal conductive traces along at least the portion of the length of the core wire, wherein the at least two longitudinal conductive traces are electrically isolated from each other and from the core wire; and
   coating a third insulative material over the second insulative material and the two circumferential arcs, around the entire circumference of the core wire along at least the portion of the length of the core wire.
Clause 15. The method of clause 14, wherein the at least two conductive traces comprise three conductive traces.
Clause 16. The method of clause 14, further comprising sintering the conductive ink.
Clause 17. The method of clause 14, wherein the conductive ink comprises particles of gold, copper, silver, or aluminum.
Clause 18. The method of clause 14, wherein at least two of the first insulative material, the second insulative material, or the third insulative material comprise the same insulative material.
Clause 19. The method of clause 14, wherein at least two of the first insulative material, the second insulative material, and the third insulative material comprise different insulative materials.
Clause 20. An intravascular sensing device, comprising:
   a guidewire configured to positioned with a blood vessel of a patient, comprising:
   a core wire;
   a first insulative coating covering at least a portion of a circumference of the core wire along at least a portion of a length of the core wire;
   at least two conductive traces comprising a sintered metallic ink, each conductive trace comprising a thickness and a width and extending longitudinally along at least the portion of the length of the core wire over an outer surface of the first insulative coating, wherein each conductive trace comprises a cross-sectional shape conforming to a curvature of the first insulative coating, wherein the at least two conductive traces and the core wire are insulated from one another;
   a second insulative coating covering an outer surface of the at least two conductive traces along at least the portion of the length of the core wire;
   a third insulative coating surrounding an entire circumference of the core wire, over a top surface of the second insulative coating along at least the portion of the length of the core wire, wherein the third insulative coating covers portions of at least one of the conductive traces, the first insulative coating, or the core wire;
   at least one of a pressure sensor or a flow sensor positioned at a distal portion of the guidewire and in electrical communication with the at least two conductive traces; and
   a connector positioned at a proximal portion of the guidewire and in electrical communication with the at least two conductive traces.

## Claims

1. An intraluminal sensing device, comprising:
a guidewire configured to be positioned within a body lumen of a patient, wherein the guidewire comprises:
a core wire;
a first insulative coating covering at least a portion of a circumference of the core wire along at least a portion of a length of the core wire;
at least two conductive traces, each comprising a thickness and a width and extending longitudinally along at least the portion of the length of the core wire over an outer surface of the first insulative coating, wherein each conductive trace comprises a cross-sectional shape conforming to a curvature of the first insulative coating, wherein the at least two conductive traces and the core wire are insulated from one another; and
a second insulative coating covering an outer surface of the at least two conductive traces along at least the portion of the length of the core wire;
a sensor configured to obtain physiological data while positioned within the body lumen, wherein the sensor is positioned at a distal portion of the guidewire and in electrical communication with the at least two conductive traces; and
a connector positioned at a proximal portion of the guidewire and in electrical communication with the at least two conductive traces.

2. The intraluminal sensing device of claim 1, further comprising a third insulative coating surrounding an entire circumference of the core wire, over a top surface of the second insulative coating along at least the portion of the length of the core wire.

3. The intraluminal sensing device of claim 2, wherein the third insulative coating covers portions of at least one of the conductive traces, the first insulative coating, or the core wire.

4. The intraluminal sensing device of claim 2, wherein the at least two conductive traces comprise three conductive traces.

5. The intraluminal sensing device of claim 2, wherein the conductive traces comprise a conductive ink.

6. The intraluminal sensing device of claim 5, wherein the conductive ink is colloidal or sintered.

7. The intraluminal sensing device of claim 2, wherein at least two of the first insulative coating, the second insulative coating, or the third insulative coating comprise the same insulative material.

8. The intraluminal sensing device of claim 2, wherein at least two of the first insulative coating, the second insulative coating, and the third insulative coating comprise different insulative materials.

9. The intraluminal sensing device of claim 2, further comprising at least two additional conductive traces, each having a thickness and a width, each running longitudinally along at least the portion of the length of the core wire over an outer surface of the second insulative coating,
wherein each conductive trace comprises a cross-sectional shape conforming to a curvature of the first insulative coating, and
wherein the at least two conductive traces, the at least two additional conductive traces, and the core wire are all mutually insulated from one another.

10. An intraluminal sensing system, comprising:
the intraluminal sensing guidewire of any one of claims 1 to 9; and
a processor circuit in communication with the intraluminal sensing guidewire, wherein the processor circuit is configured to receive physiology data obtained by the sensor, process the physiology data, and output a graphical representation of the physiology data to a display in communication with the processing system.

11. A method for manufacturing an intraluminal sensing guidewire, comprising:
providing a core wire;
coating a first insulative material around an entire circumference of the core wire along at least a portion of a length of the core wire;
coating a conductive ink over the first insulative material around the entire circumference of the core wire along at least the portion of the length of the core wire;
coating a second insulative material over the conductive ink around the entire circumference of the core wire along at least the portion of the length of the core wire;
removing material from at least two circumferential arcs of at least the second insulative material and the conductive ink, such that the conductive ink forms at least two longitudinal conductive traces along at least the portion of the length of the core wire, wherein the at least two longitudinal conductive traces are electrically isolated from each other and from the core wire; and
coating a third insulative material over the second insulative material and the two circumferential arcs, around the entire circumference of the core wire along at least the portion of the length of the core wire.

12. The method of claim 11, further comprising sintering the conductive ink.

13. The method of claim 11, wherein at least two of the first insulative material, the second insulative material, or the third insulative material comprise the same insulative material.

14. The method of claim 11, wherein at least two of the first insulative material, the second insulative material, and the third insulative material comprise different insulative materials.

15. An intravascular sensing device, comprising:
a guidewire configured to positioned with a blood vessel of a patient, comprising:
a core wire;
a first insulative coating covering at least a portion of a circumference of the core wire along at least a portion of a length of the core wire;
at least two conductive traces comprising a sintered metallic ink, each conductive trace comprising a thickness and a width and extending longitudinally along at least the portion of the length of the core wire over an outer surface of the first insulative coating, wherein each conductive trace comprises a cross-sectional shape conforming to a curvature of the first insulative coating, wherein the at least two conductive traces and the core wire are insulated from one another;
a second insulative coating covering an outer surface of the at least two conductive traces along at least the portion of the length of the core wire;
a third insulative coating surrounding an entire circumference of the core wire, over a top surface of the second insulative coating along at least the portion of the length of the core wire, wherein the third insulative coating covers portions of at least one of the conductive traces, the first insulative coating, or the core wire;
at least one of a pressure sensor or a flow sensor positioned at a distal portion of the guidewire and in electrical communication with the at least two conductive traces; and
a connector positioned at a proximal portion of the guidewire and in electrical communication with the at least two conductive traces.
